# EUROPEAN PATENT SPECIFICATION

(11) **EP 2 233 133 B1**
(45) Date of publication and mention of the grant of the patent: **25.07.2012**
(21) Application number: 10156610.7
(22) Date of filing: 16.03.2010
(51) Int. Cl.: A61K 9/20, A61K 31/505, A61K 47/02, A61K 47/10, A61K 9/28

(54) **Stable Rosuvastatin Compositions**
Stabile Rosuvastatin Zubereitungen
Compositions stables comprenant de Rosuvastatine

(30) Priority: 17.03.2009 TR 200902077
(43) Date of publication of application: 29.09.2010
(73) Proprietor: Sanovel Ilaç Sanayi Ve Ticaret Anonim Sirketi, 34460 Sariyer/Istanbul (TR)
(72) Inventor: Sezgin, Asiye, 34398, Istanbul (TR); Güler, Nehir, 34398, Istanbul (TR); Türkyilmaz, Ali, 34398, Istanbul (TR); Cifter, Ümit, 34398, Istanbul (TR); Öner, Levent, Ankara (TR); Cakir, Fatih, 34398, Istanbul (TR)
(74) Representative: Sevinç, Erkan

(56) References cited:
- WO-A-01/54669
- ANONYMOUS: "Crestor Leaflet" INTERNET ARTICLE, [Online] October 2008 (2008-10), pages 1-14, XP002548139 Retrieved from the Internet: URL:http://xpil.medicines.org.uk/ViewPil.a spx?DocID=11972> [retrieved on 2009-09-30]

## Description

### Technical Aspect

The present invention is relates to novel stable pharmaceutical compositions of rosuvastatin calcium, comprising dicalcium phosphate anhydrous in core and polyethylene glycol in combination with titanium dioxide in coating which is free of ferric oxide. More specifically pharmaceutical compositions of rosuvastatin calcium comprise the raito of dicalcium phosphate anhydrous to the polyethylene glycol in combination with titanium dioxide in the range of between 20:1 to 1:10 by weight.

### Background of the Invention

Rosuvastatin calcium is a synthetic lipid-lowering agent which is a selective and competitive inhibitor of HMG-CoA reductase inhibitor. Its chemical name is bis[(E)-7-[4-(4-fluorophenyl)-6-isopropyl-2-[methyl(methylsulfonyl)amino]pyrimidin-5-yl]-(3R,5S)-dihydroxyhept-6-enoic acid] calcium salt and its chemical structure is shown in the Formula I.

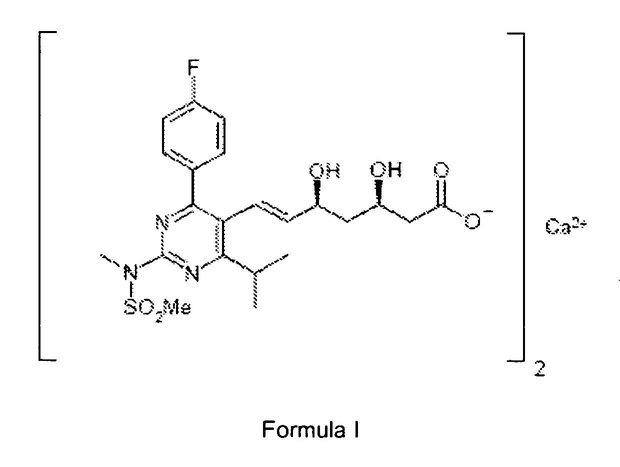

Rosuvastatin calcium is marketed under the brand name CRESTOR^{®} and it is administered in a daily dose of from about 5 mg to about 40 mg for the treatment of hyperlipoproteinemia, hypercholesterolemia and atherosclerosis.

Rosuvastatin and the synthesis of rosuvastatin calcium was first described in patent EP-B-521 471, Shionogi, 01.07.1991.

It is known that HMG-CoA reductase inhibitors such as rosuvastatin calcium is unstable under conditions of acidity, oxygen, light and humidty. Rosuvastatin calcium is degraded to [(E)-7-[4-(4-fluorophenyl)-6-isopropyl-2-[methyl(methylsulfonyl)amino]pyrimidin-5-yl]-(3R,5S)3,5-dihydroxyhept-6-enoicacid)-3,6) lactone (hereinafter reffered to as "lactone"), an oxidation product and when exposed to light, it undergoes to some other isomers.

The problem underlying the present invention is to provide a stabilised pharmaceutical composition of rosuvastatin calcium, comprising dicalcium phosphate anhydrous which is stable throughout the shelflife, methods for their preparation, and methods for treatment using the same.

In prior art, there are many patents including rosuvastatin with improved stability, for example in EP patent no EP-B-1 223 918, AstraZeneca AB, 26.01.2000 discloses a pharmaceutical composition comprising rosuvastatin, wherein the stability of rosuvastatin is improved, with an inorganic salt in which a cation is multivalent, preferably tribasic calcium phosphate.

PCT application no WO 01/54669 A1, Astrazeneca UK Limited, 26.01.2000 discloses that it is not sufficient to improve stability by solely controlling pH in the formulation and suggests the use of inorganic salt in the pharmaceutical composition of rosuvastatin in which the cation is multivalent and the multivalent cation is not synthetic hydrotalcite.

Another PCT application no WO 2007/071357, Lek Pharmaceuticals D.D., 20.12.2005 discloses a pharmaceutical composition comprising rosuvastatin or a pharmaceutically acceptable salt thereof **characterized in that** containing less than 0.5% lactone and less than 0.05% oxidation product as measured by HPLC.

PCT application no WO 2008/062476, Glenmark Pharmaceutical Limited, 31.10.2006 discloses a pharmaceutical composition comprising a therapeutically effective amount of a HMG-CoA reductase inhibitor which is rosuvastatin and an inorganic salt of a monovalent cation wherein the pH of the composition is less than or equal to about 8.0, and free of an alkaline stabilizer.

The sensitivity of an active substance for stability throughout the shelflife also depends on a pharmaceutical dosage form and pharmaceutical excipients in it. Therefore, there is need in the art for pharmaceutical compositions of rosuvastatin calcium with improved stability against its degredation products such as lactones, oxidation products and other isomers.

### Description of the invention

It is known from the prior art that rosuvastatin calcium has a problem associated with its degradation under certain conditions such as acidity, oxygen, light and humidty. This makes it difficult to formulate a pharmaceutical product and provide a pharmaceutical composition wich is stable troughout the shelflife. The major degradation product formed is lactone.

Accordingly, it is important to find a pharmaceutical composition of rosuvastatin calcium which remains stable over a prolonged period. We have now discovered a novel stable pharmaceutical composition of rosuvastatin calcium which has advantageous properties and which solves one or more of the problems in prior art associated with formulation of the rosuvastatin calcium.

The main embodiment of the invention comprises novel stable pharmaceutical compositions of rosuvastatin calcium, comprising dicalcium phosphate anhydrous in core and polyethylene glycol in combination with titanium dioxide in coating which is free of ferric oxide.

Surprisingly we have found that when the weight ratio of dicalcium phosphate anhydrous to polyethylene glycol in combination with titanium dioxide is between 20:1 to 1:10 by weight, it reduces the formation of the degredation product, lactone throughout the shelflife.

Preferably the ratio of dicalcium phosphate anhydrous to the polyethylene glycol in combination with titanium dioxide is in the range of between 16:1 to 14:1 by weight.

The pharmaceutical compositions of the invention include oral dosage forms such as tablets, capsules, etc. Preferably the pharmaceutical composition of the invention is formulated as a tablet. Accordingly a further embodiment of the invention comprises a pharmaceutical composition comprising rosuvastatin calcium and a dicalcium phosphate anhydrous, and one or more fillers, binders, disintegrants or lubricants.

The pharmaceutical compositions of the present invention may contain one or more pharmaceutically acceptable excipients. Suitable pharmaceutically acceptable excipients include, but are not limited to, diluents, binders, disintegrants, surface active agents, glidants, lubricants and the like and mixtures thereof.

Suitable fillers may include but not limited to lactose, sugar, starches, modified starches, mannitol, calcium sulfate, xylitol and the like and mixtures thereof. In this present invention the most preffered filler is lactose.

Suitable binders may include but not limited to microcrystalline cellulose, sugars, wax binders, methylcellulose, carboxymethylcellulose, hydroxypropyl methylcellulose, hydroxyethyl cellulose, hydroxypropyl cellulose, gelatin and sodium alginate and the like and mixtures thereof. In this present invention the most preffered binder is microcrystalline cellulose.

Suitable disintegrants may include but not limited to crospovidone, crosscarmellose sodium, polyvinylpyrrolidone, sodium starch glycollate and the like and mixtures thereof. In this present invention the most preffered disintegrant is crospovidone.

Suitable lubricants may include but not limited to magnesium stearate, stearic acid, calcium stearate, talc, sodium stearyl fumarate and the like and mixtures thereof. In this present invention the most preffered lubricant is magnesium stearate.

The pharmaceutical compositions according to the present invention may further comprise one or more pharmaceutically acceptable preservatives in coating. Suitable preservatives include, but are not limited to methyl paraben, propyl paraben, sodium benzoate and benzoic acid. Most preffered preservatives are methyl paraben and propyl paraben.

Preferred pharmaceutical composition of the present invention is a tablet formulation and it is consisting of a core which includes 1 to 15% rosuvastatin, 15 to 20% dicalcium phosphate anhydrous, 5 to 10% crospovidone, 15 to 25% microcrystalline cellulose, 30 to 60% lactose and 0.5 to 5% magnesium stearate by weight and this tablet formulation has a coating.

The weight gain provided by the coating is between 1 to 5% (w/w) which is consisting of 40 to 70% hydroxypropyl methylcellulose (HPMC) and 5 to 70% polyethylene glycol in combination with titanium dioxide by weight. The amount of polyethylene glycol in combination with titanium dioxide is present between 30 to 60% by weight of the coating. Preferably it is between 35 to 40% by weight.

The pharmaceutical composition of the present invention can be prepared, using standard techniques and manufacturing processes well known in the art, such as wet and dry granulation or direct compression. Preferably direct compression is used in this invention.

One of the main embodiments of this present invention is directed to tablet coating; it can be applied, for example by spray-coating with a water-based film coating formulation. The coating comprises, for example, hydroxypropyl methylcellulose, polyethylene glycol and titanium dioxide but free of ferric oxide.

The pharmaceutical compositions of the present invention may be formulated for administration to mammals such as humans or animals for treating hyperlipoproteinemia, hypercholesterolemia and atherosclerosis.

This invention is further defined by reference to the following examples. Although the example is not intended to limit the scope of the present invention, it should be considered in the light of the description detailed above. It will be apparent to those skilled in the art that many modifications, both to materials and methods, may be practiced without departing from the scope of the invention.

**Example 1 : pharmaceutical composition of rosuvastatin calcium**

| **Ingredients** | **Amount (mg/tablet)** |
|---|---|
| *core* | |
| Rosuvastatin calcium | 20.8 |
| Dicalcium phosphate anhydrous | 51.6 |
| Crospovidone | 25.0 |
| Microcrystalline cellulose | 62.0 |
| Lactose anhydrous | 136.8 |
| Magnesium stearate | 3.8 |

| *coating* | |
|---|---|
| HPMC | 5.625 |
| polyethylene glycol and titanium dioxide | 3.375 |

We made stability and some stress tests to the tablet compositions of rosuvastatin calcium which are coated according to the present invention.

The coated tablets were tested against light-stress test according to the method described in ICH guidelines. After the test we observed no change in colour of tablet compositions. By comparison a similar formulation in which the tablets were not coated was tested against light-stress test. After the test the tablets without coating turned out to yellowish colour which shows that the coating of this present invention protects the tablets degrading from the light.

We also measured the lactones and total impurities formed after the light-stress test. The core tablets have 0.21 % w/w lactone but the tablets with coating have only 0.08% w/w lactone. The total impurities may also includes some other degredation products occurred by light and oxidation. The total impurity of the core tablets is found to be 4.71% w/w and the total impurity of the tablets with coating is only 0.70% w/w.

Tablets with coating were also stored at 70°C/80% relative humidity for one week. After one week lactone was found to be only 0.14% w/w and by comparison we stored the tablets without blisters to see the result of oxidation and only 0.38% w/w lactone was formed.

According to the stability test results of these tablet compositions of the present invention, the maximum amount of lactone is found to be 0.08% w/w after 1 year at 25°C/60%RH and 0.28% w/w after 6 months at 40°C/75%RH.

## Claims

1. A pharmaceutical composition of rosuvastatin calcium, comprising dicalcium phosphate anhydrous in core and polyethylene glycol in combination with titanium dioxide in coating which is free of ferric oxide.

2. The pharmaceutical composition of rosuvastatin calcium according to claim 1,
wherein the ratio of dicalcium phosphate anhydrous to the polyethylene glycol in combination with titanium dioxide is in the range of between 20:1 to 1:10 by weight.

3. The pharmaceutical composition of rosuvastatin calcium according to claim 2,
wherein the preffered ratio of dicalcium phosphate anhydrous to the polyethylene glycol in combination with titanium dioxide is in the range of between 16:1 to 14:1 by weight.

4. The pharmaceutical composition of rosuvastatin calcium according to claims 1 to 3, wherein polyethylene glycol in combination with titanium dioxide is present in an amount of 30 to 60 % by weight of the coating.

5. The pharmaceutical composition of rosuvastatin calcium according to claim 4,
wherein polyethylene glycol in combination with titanium dioxide is preferably present in an amount of 35 to 40 % by weight of the coating.

6. The pharmaceutical composition of rosuvastatin calcium according to claims 1 to 5, pharmaceutical dosage form of the composition is tablet.

7. The pharmaceutical composition of rosuvastatin calcium according to claims 1 to 6, consisting of
a. the core, consisting of
a. 1 to 15% rosuvastatin
b. 15 to 20 % dicalcium phosphate anhydrous
c. 5 to 10 % crospovidone
d. 15 to 25% microcrystalline cellulose
e. 30 to 60 % lactose
f. 0.5 to 5% magnesium stearate
b. the coating, consisting of
a. 40 to 70% HPMC
b. 5 to 60 % polyethylene glycol and titanium dioxide

8. The pharmaceutical composition of rosuvastatin calcium according to any preceding claims, further comprising preservatives in coating.

9. The pharmaceutical composition of rosuvastatin calcium according to claim 8, wherein the preservatives are selected from the group comprising methyl paraben, propyl paraben, sodium benzoate and benzoic acid.

10. The pharmaceutical composition of rosuvastatin calcium according to claim 8, wherein the preservatives are preferably methyl paraben and propyl paraben.

## Patentansprüche

1. Pharmazeutische Rosuvastatincalciumzusammensetzung, welche anhydrisches Dicalciumphosphat in einem Kern und Polyethylenglykol in Kombination mit Titandioxid in einer Beschichtung, welche frei von Eisenoxid ist, umfasst.

2. Pharmazeutische Rosuvastatincalciumzusammensetzung gemäß Anspruch 1, wobei das Gewichtsverhältnis von anhydrischem Dicalciumphosphat zu dem Polyethylenglykol in Kombination mit Titandioxid in dem Bereich zwischen 20:1 und 1:10 ist.

3. Pharmazeutische Rosuvastatincalciumzusammensetzung gemäß Anspruch 2, wobei das bevorzugte Gewichtsverhältnis von anhydrischem Dicalciumphosphat zu dem Polyethylenglykol in Kombination mit Titandioxid in dem Bereich zwischen 16:1 und 14:1 ist.

4. Pharmazeutische Rosuvastatincalciumzusammensetzung gemäß einem der Ansprüche 1 bis 3, wobei Polyethylenglykol in Kombination mit Titandioxid in einer Menge von 30 bis 60 Gew.-% der Beschichtung vorliegt.

5. Pharmazeutische Rosuvastatincalciumzusammensetzung gemäß Anspruch 4, wobei Polyethylenglykol in Kombination mit Titandioxid vorzugsweise in einer Menge von 35 bis 40 Gew.-% der Beschichtung vorliegt.

6. Pharmazeutisch Rosuvastatincalciumzusammensetzung gemäß einem der Ansprüche 1 bis 5, wobei die pharmazeutische Darreichungsform der Zusammensetzung eine Tablette ist.

7. Pharmazeutische Rosuvastatincalciumzusammensetzung gemäß einem der Ansprüche 1 bis 6, welche aus Folgendem besteht:
a. dem Kern, welcher aus Folgendem besteht:
a. 1 bis 15 % Rosuvastatin
b. 15 bis 20 % anhydrisches Dicalciumphosphat
c. 5 bis 10 % Crospovidon
d. 15 bis 25 % mikrokristalline Zellulose
e. 30 bis 60 % Laktose
f. 0,5 bis 5 % Magnesiumstereat
b. der Beschichtung, welche aus Folgendem besteht:
a. 40 bis 70 % HPMC
b. 5 bis 60 % Polyethylenglykol und Titandioxid.

8. Pharmazeutische Rosuvastatincalciumzusammensetzung gemäß einem der vorhergehenden Ansprüche, welche weiter Konservierungsmittel in der Beschichtung umfasst.

9. Pharmazeutische Rosuvastatincalciumzusammensetzung gemäß Anspruch 8, wobei die Konservierungsmittel ausgewählt sind aus der Gruppe, welche Methylparaben, Propylparaben, Natriumbenzoat und Benzolsäure umfasst.

10. Pharmazeutische Rosuvastatincalciumzusammensetzung gemäß Anspruch 8, wobei die Konservierungsmittel vorzugsweise Methylparaben und Propylparaben sind.

## Revendications

1. Composition pharmaceutique de rosuvastatine calcique, comprenant du phosphate dicalcique anhydre dans un noyau et du polyéthylène glycol en association avec du dioxyde de titane dans un enrobage qui est exempt d'oxyde de fer.

2. Composition pharmaceutique de rosuvastatine calcique selon la revendication 1, dans laquelle le rapport entre le phosphate dicalcique anhydre et le polyéthylène glycol en association avec du dioxyde de titane se situe dans la plage entre 20/1 et 1/10 en poids.

3. Composition pharmaceutique de rosuvastatine calcique selon la revendication 2, dans laquelle le rapport préféré entre le phosphate dicalcique anhydre et le polyéthylène glycol en association avec du dioxyde de titane se situe dans la plage entre 16/1 et 14/1 en poids.

4. Composition pharmaceutique de rosuvastatine calcique selon les revendications 1 à 3, dans laquelle le polyéthylène glycol en association avec du dioxyde de titane est présent en une quantité de 30 à 60 % en poids de l'enrobage.

5. Composition pharmaceutique de rosuvastatine calcique selon la revendication 4, dans laquelle le polyéthylène glycol en association avec du dioxyde de titane est de préférence présent en une quantité de 35 à 40 % en poids de l'enrobage.

6. Composition pharmaceutique de rosuvastatine calcique selon les revendications 1 à 5, la forme galénique pharmaceutique de la composition étant le comprimé.

7. Composition pharmaceutique de rosuvastatine calcique selon les revendications 1 à 6, constituée par
a. le noyau, constitué par
a. 1 à 15 % de rosuvastatine
b. 15 à 20 % de phosphate dicalcique anhydre
c. 5 à 10 % de crospovidone
d. 15 à 25 % de cellulose microcristalline
e. 30 à 60 % de lactose
f. 0,5 à 5 % de stéarate de magnésium
b. l'enrobage, constitué par
a. 40 à 70 % d'HPMC
b. 5 à 60 % de polyéthylène glycol et de dioxyde de titane.

8. Composition pharmaceutique de rosuvastatine calcique selon l'une quelconque des revendications précédentes, comprenant en outre des conservateurs dans l'enrobage.

9. Composition pharmaceutique de rosuvastatine calcique selon la revendication 8, dans laquelle les conservateurs sont sélectionnés dans le groupe comprenant le parahydroxybenzoate de méthyle, le parahydroxybenzoate de propyle, le benzoate de sodium et l'acide benzoïque.

10. Composition pharmaceutique de rosuvastatine calcique selon la revendication 8, dans laquelle les conservateurs sont de préférence le parahydroxybenzoate de méthyle et le parahydroxybenzoate de propyle.
